# EUROPEAN PATENT APPLICATION

(11) **EP 4 610 268 A1**
(43) Date of publication of application: **03.09.2025**
(21) Application number: 23882695.2
(22) Date of filing: 25.10.2023
(51) Int. Cl.: C07K 5/08, C12N 15/11, C12N 15/87

(54) **CELL-MEMBRANE-PERMEABLE PEPTIDE FOR CONJUGATION**

(30) Priority: 25.10.2022 JP 2022170448
(71) Applicant: AGC INC., Chiyoda-ku, Tokyo 1008405 (JP); The University of Tokyo, Bunkyo-ku, Tokyo 113-8654 (JP)
(72) Inventor: OTA, Yu, Tokyo 100-8405 (JP); OKAZOE, Takashi, Tokyo 100-8405 (JP); AIKAWA, Kohsuke, Tokyo 113-8654 (JP); KOHATA, Ai, Tokyo 113-8654 (JP); SANDO, Shinsuke, Tokyo 113-8654 (JP); MORIMOTO, Jumpei, Tokyo 113-8654 (JP); KADOTA, Koji, Tokyo 113-8654 (JP)
(74) Representative: Müller-Boré & Partner Patentanwälte PartG mbB
(86) International application number: PCT/JP2023/038590
(87) International publication number: WO 2024/090491

(57) **Abstract**

The present invention provides a cell membrane-penetrating peptide for conjugation, represented by the following general formula (P1):

R¹⁰¹-S-S-(Z¹²)q₂-A¹ (P1)

wherein R¹⁰¹ is a substituted or unsubstituted 2-pyridyl group; A¹ is a cell membrane-penetrating peptide; Z¹² is a divalent organic group; and q2 is 0 or 1, wherein the cell membrane-penetrating peptide is a peptide with two or more peptide-bonded amino acids and with at least one of amino acid residues constituting the peptide having, in its side chain, a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms.

## Description

### TECHNICAL FIELD

The present invention relates to a cell membrane-penetrating peptide for conjugation that has excellent cell penetration and, when bound to a nucleic acid, can efficiently introduce the nucleic acid into a cell.

Priority is claimed on Japanese Patent Application No. 2022-170448, filed October 25, 2022, the contents of which are incorporated herein by reference.

### BACKGROUND ART

In recent years, there have been ongoing researches on nucleic acid drugs using oligonucleotides. Nucleic acid drugs have the advantages of being highly specific to target molecules and having less side effects. However, nucleic acid drugs have low cell penetration, and hence are difficult to deliver to target molecules present in cells. In particular, siRNA is double-stranded, and has a larger molecular weight and negative charge than an antisense RNA. As a result, siRNA has inferior cell penetration to an antisense RNA, which necessitates drug delivery by a carrier. Drug delivery agents that use lipid nanoparticles (Patent Document 1) and cationic polymer nanoparticles (Patent Document 2) are known. However, many improvements are needed in terms of the efficiency of cell penetration and the toxicity concerns.

On the other hand, a compound having a polyfluoro structure is known to be stable and is less toxic in the body and is excellent in uptake by the cells and transport out of endosomes (Non-Patent Document 1). Taking advantage of such nature, studies are being conducted on introducing a polyfluoro structure into oligonucleotides or peptide nucleic acids as a moiety capable of penetrating cell membranes (Patent Documents 3 and 4).

Further, introduction of polyfluoro structures into peptides is also implemented. For example, it has been reported that a peptide dendrimer using lysine having an amino acid group as its side chain perfluoroacylated as the constituting amino acid, can be used to deliver the genes (Non-Patent Document 2).

### Citation List

### Patent Document

Patent Document 1 : International Patent Application Publication No. 2011/036557
Patent Document 2 : International Patent Application Publication No. 2017/212006
Patent Document 3 : International Patent Application Publication No. 2012/130941
Patent Document 4 : International Patent Application Publication No. 2021/060506

### Non-Patent Document

Non-Patent Document 1: Zhang et al., MRS Communications, 2018, vol.8, p.303-313.
Non-Patent Document 2: Cai et al., ACS Applied Materials and Interfaces, 2016, vol.8, p.5821-5832.
Non-Patent Document 3: Murayama et al., Chemistry A European Journal, 2013, vol.19, p.14151-14158.

### SUMMARY OF THE INVENTION

### Technical Problem

The present invention aims to provide a cell membrane-penetrating peptide which is a peptide that can be conjugated to a subject nucleic acid to be introduced into a cell, improves the cell penetration of the nucleic acid, and does not impair the function of the nucleic acid within the cell, as well as a nucleic acid for transfection conjugated with the cell membrane-penetrating peptide.

### Solution to Problem

The present inventors have conducted studies to produce a peptide containing an amino acid residue having a fluoroalkyl group introduced into its side chain and as a result, found that such a peptide is excellent in cell penetration. Further, the inventors have found that by linking the peptide via a disulfide bond to the subject nucleic acid to be introduced into a cell (introduction subject nucleic acid), the nucleic acid can be imparted with high cell penetration without impairing its function within the cell, thus completing the present invention.

That is, the present invention is as follows.
[1] A cell membrane-penetrating peptide for conjugation, represented by the following general formula (P1):

   [Chem. 1] R¹⁰¹-S-S- (Z¹²)q₂-A¹ (P1)

   wherein R¹⁰¹ is a substituted or unsubstituted 2-pyridyl group; A¹ is a cell membrane-penetrating peptide; Z¹² is a divalent organic group; and q₂ is 0 or 1,
   wherein the cell membrane-penetrating peptide is a peptide with two or more peptide-bonded amino acids and with at least one of amino acid residues constituting the peptide having, in its side chain, a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms.
[2] The cell membrane-penetrating peptide according to [1], wherein the side chain having the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is a group represented by the following general formula (f-1) or (f-2): 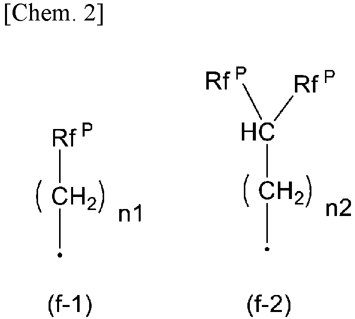 wherein Rf^{P} is a fully halogenated C₁₋₁₀ alkyl group containing at least two fluorine atoms, or a group having 1 to 5 etheric oxygen atoms between carbon atoms of a fully halogenated C₂₋₁₀ alkyl group containing at least two fluorine atoms, n1 is an integer of 0 to 10, n2 is an integer of 0 to 9, and a black dot represents a bonding site.
[3] The cell membrane-penetrating peptide according to [1] or [2], wherein the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is or is not further substituted with a halogen atom other than a fluorine atom.
[4] The cell membrane-penetrating peptide according to any one of [1] to [3], wherein a C-terminal or an N-terminal of the cell membrane-penetrating peptide is or is not protected with a protecting group.
[5] The cell membrane-penetrating peptide according to any one of [1] to [4], wherein the Z¹² is an alkylene group having 1 to 10 carbon atoms, an alkenylene group having 1 to 10 carbon atoms, an oxygen atom (-O-), a sulfur atom (-S-), -NH-, -N(CH₃)-, -N(C₂H₅)-, - N(C₃H₇)-, -C(=O)-, -S(=O)₂-, a poly(ethylene glycol) group (PEG: -(C₂ H₄ O)n-), a siloxane bond, a silyl ether bond, a group resulting from abstraction of two hydrogen atoms from a cycloalkane, a group resulting from abstraction of two hydrogen atoms from an aromatic ring, a group resulting from abstraction of two hydrogen atoms from a heterocycle, a sugar, or a group in which two or more of these are combined.
[6] A method for producing a nucleic acid for transfection, comprising binding the cell membrane-penetrating peptide of any one of [1] to [5] to a subject nucleic acid to be introduced into a cell to produce a nucleic acid for transfection, wherein the subject nucleic acid is represented by the following general formula (P2):

   [Chem. 3] B¹-(Z¹¹)q₁-SH (P2)

   wherein B¹ is a nucleic acid; Z¹¹ is a divalent organic group; q₁ is 0 or 1, and the nucleic acid for transfection is represented by the following general formula (P3):

   [Chem. 4] B¹-(Z¹¹)q₁-S-S-(Z¹²)q₂-A¹ (P3)

   wherein A¹, Z¹², and q₂ are the same as in the general formula (P1); and B¹, Z¹¹, and q₁ are the same as in the general formula (P2).
[7] A nucleic acid for transfection, comprising a subject nucleic acid to be introduced into a cell and a cell membrane-penetrating peptide that are linked via a linking group, wherein
   the linking group contains at least one disulfide bond, and
   the cell membrane-penetrating peptide is a peptide with two or more peptide-bonded amino acids and with at least one of amino acid residues constituting the peptide having, in its side chain, a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms.
[8] The nucleic acid for transfection according to [7], wherein the side chain having the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is a group represented by the following general formula (f-1) or (f-2): 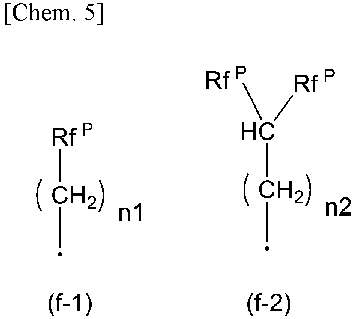 wherein Rf^{P} is a fully halogenated C₁₋₁₀ alkyl group containing at least two fluorine atoms, or a group having 1 to 5 etheric oxygen atoms between carbon atoms of a fully halogenated C₂₋₁₀ alkyl group containing at least two fluorine atoms, n1 is an integer of 0 to 10, n2 is an integer of 0 to 9, and a black dot represents a bonding site.
[9] The nucleic acid for transfection according to [7] or [8], wherein a C-terminal or an N-terminal of the cell membrane-penetrating peptide is or is not protected with a protecting group.
[10] A method for transfection with a nucleic acid, comprising contacting the nucleic acid for transfection of any one of [7] to [9] with a cell to introduce the nucleic acid into the cell.

### Advantageous Effects of Invention

The cell membrane-penetrating peptide for conjugation according to the present invention includes a cell membrane-penetrating peptide portion and a linking group portion having a disulfide bond. Therefore, by conjugating the cell membrane-penetrating peptide for conjugation according to the present invention to an introduction subject nucleic acid, a nucleic acid for transfection can be produced in which the cell membrane-penetrating peptide is linked to the introduction subject nucleic acid via a disulfide bond.

The nucleic acid for transfection has excellent cell penetration due to the cell membrane-penetrating peptide, but is allowed to be present inside the cell in a state of being cleaved from the peptide portion. Therefore, the nucleic acid for transfection not only has high cell penetration but also can exert its functions within the cell to the same extent as the nucleic acid before modification with the peptide, so that the nucleic acid for transfection is extremely useful as a nucleic acid carrier into cells.

### BRIEF DESCRIPTION OF THE DRAWINGS

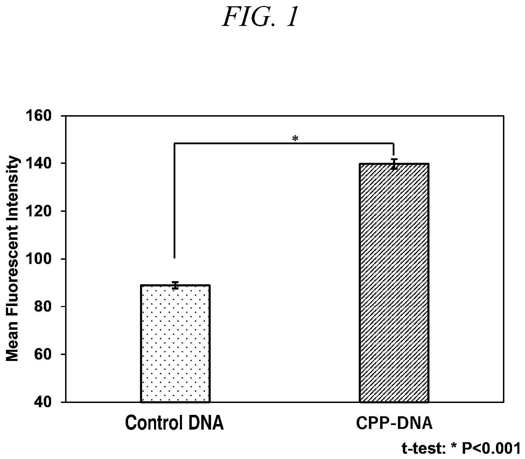

[FIG. 1] A diagram showing comparison of the average fluorescence intensities in flow cytometry of HeLa cells treated for 4 hours at 37°C in a sample solution containing FAM-modified nucleic acid (control-DNA) or FAM-modified nucleic acid modified with a cell membrane-penetrating peptide (CPP-DNA) in Test Example 1.

### DESCRIPTION OF EMBODIMENTS

In the present invention and the present specification, the term "nucleic acid" refers to a molecule in which nucleotides are linked by phosphodiester bonds. Such nucleotides encompass not only natural nucleotides (naturally occurring nucleotides) such as those included in DNA and RNA, but also artificial nucleotides that are obtained by modifying natural nucleotides and can form phosphodiester bonds with natural nucleotides. Examples of artificial nucleotides include natural nucleotides with their side chains etc. modified with functional groups such as amino groups, nucleotides in which the hydroxyl group at the 2' position of the ribose backbone is replaced with a methoxy group, a fluoro group, a methoxyethyl group or the like, phosphorothioate nucleotides (in which the oxygen atom of the phosphate group is replaced with a sulfur atom), morpholino nucleotides (in which ribose or deoxyribose is replaced with a morpholine ring), BNA (Bridged Nucleic Acid), HNA (Hexitol Nucleic Acid), LNA (Locked Nucleic Acid), PNA (Peptide Nucleic Acid), TNA (Threse Nucleic Acid), GNA (Glycerol Nucleic Acid), CeNA (Cyclohexenyl Nucleic Acid) and the like (Non-Patent Document 3). Further, the term "nucleic acid" encompasses molecules such as DNA and RNA in which only one or more types of naturally occurring nucleotides are bonded via phosphodiester bonds, molecules in which one or more types of naturally occurring nucleotides and one or more types of artificial nucleotides are bonded together via phosphodiester bonds, and molecules in which only one or more types of artificial nucleotides are bonded via phosphodiester bonds.

In the present invention and the present specification, "Cₚ₁₋ₚ₂" (p1 and p2 are positive integers which satisfy p1 < p2) means a group having p1 to p2 carbon atoms.

In the present invention and the present specification, a "C₁₋₁₀ alkyl group" means an alkyl group having 1 to 10 carbon atoms, which may be linear or branched. A "C₂₋₁₀ alkyl group" means an alkyl group having 2 to 10 carbon atoms, which may be linear or branched. Examples of the C₁₋₁₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group and a decyl group.

In the present invention and the present specification, a "C₁₋₃₀ alkyl group" is an alkyl group having 1 to 30 carbon atoms, which may be linear or branched. A "C₂₋₃₀ alkyl group" is an alkyl group having from 2 to 30 carbon atoms, which may be linear or branched. Examples of the C₁₋₃₀ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a tricosyl group, a tetracosyl group, a pentacosyl group, a hexacosyl group, a heptacosyl group, an octacosyl group, a nonacosyl group and a triacontyl group.

In the present invention and the present specification, a "C₁₋₆ alkyl group" is an alkyl group having 1 to 6 carbon atoms, which may be linear or branched. Examples of the C₁₋₆ alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group and a hexyl group.

In the present invention and the present specification, a "C₆₋₁₄ aryl group" means an aromatic hydrocarbon group having 6 to 14 carbon atoms and is particularly preferably a C₆₋₁₂ aryl group. Examples of the C₆₋₁₄ aryl group include a phenyl group, a naphthyl group, an anthryl group and a 9-fluorenyl group, and a phenyl group is particularly preferable.

In the present invention and the present specification, a "C₆₋₁₄ aryl group which may be substituted" is a group having one or more, preferably 1 to 3, hydrogen atoms bonded to a carbon atom of a C₆₋₁₄ aryl group replaced with another functional group. When the aryl group has two or more substituents, the substituents may be of the same type or different types. Examples of the substituent include a nitro group, a halogen atom (a fluorine atom, a chlorine atom, a bromine atom or an iodine atom), a C1-6 alkyl group, a C₁₋₆ alkoxy group, and a methylenedioxy group (-O-CH₂-O-). Examples of the "C₆₋₁₄ aryl group which may be substituted" include a phenyl group, a naphthyl group, an anthryl group, a 4-nitrophenyl group, a 4-methoxyphenyl group, a 2,4-dimethoxyphenyl group, a 3,4-dimethoxyphenyl group, a 4-methylphenyl group, a 2,6-dimethylphenyl group, a 3-chlorophenyl group and a 1,3-benzodioxol-5-yl group.

In the present invention and the present specification, a "C₆₋₁₄ aryl-C₁₋₆ alkyl group" is a group having one hydrogen atom bonded to a carbon atom of a C₁₋₆ alkyl group replaced with a C₆₋₁₄ aryl group. Examples of the C₆₋₁₄ aryl group in the C₆₋₁₄ aryl-C₁₋₆ alkyl group include a phenyl group, a naphthyl group, an anthryl group and a 9-fluorenyl group, and particularly preferable is a phenyl group or a 9-fluorenyl group. The C₁₋₆ alkyl group in the C₆₋₁₄ aryl-C₁₋₆ alkyl group is preferably a C₁₋₄ alkyl group. Examples of the C₆₋₁₄ aryl-C₁₋₆ alkyl group include a benzyl group, a diphenylmethyl group, a triphenylmethyl group, a 2-phenylethyl group, a 9-anthrylmethyl group and a 9-fluorenylmethyl group.

In the present invention and the present specification, a "halogen atom" means a fluorine atom, a chlorine atom, a bromine atom or an iodine atom. A "halogen atom other than a fluorine atom" means a chlorine atom, a bromine atom or an iodine atom. The "halogen atom other than a fluorine atom" is preferably a chlorine atom or a bromine atom, particularly preferably a chlorine atom.

In the present invention and the present specification, a "C₁₋₆ alkoxy group" means a group having an oxygen atom bonded to the bond terminal of a C₁₋₆ alkyl group having 1 to 6 carbon atoms. The C₁₋₆ alkoxy group may be linear or branched. Examples of the C₁₋₆ alkoxy group include a methoxy group, an ethoxy group, a propoxy group, a butoxy group, a tert-butoxy group, a pentyloxy group and a hexyloxy group.

In the present invention and the present specification, an "etheric oxygen atom" means an oxygen atom linking carbon atoms, and does not include oxygen atoms directly bonded in series. The number of etheric oxygen atom which an alkyl group having Nc (Nc is an integer of 2 or more) carbon atoms may have is Nc-1 at the most. Hereinafter, "an alkyl group having an etheric oxygen atom between carbon atoms" may be referred to as "an ether-bond-containing alkyl group".

In the present invention and the present specification, "a C₁₋₃₀ alkyl group substituted with at least two fluorine atoms (when the C₁₋₃₀ alkyl group has two or more carbon atoms, it may have 1 to 5 etheric oxygen atoms between the carbon atoms)" means "a C₁₋₃₀ alkyl group substituted with at least two fluorine atoms, or a group having 1 to 5 etheric oxygen atoms between the carbon atoms of a C₂₋₃₀ alkyl group substituted with at least two fluorine atoms".

Further, in the following descriptions, "compound n" means a compound represented by the formula (n).

### <<Cell membrane-penetrating peptide for conjugation>>

The cell membrane-penetrating peptide for conjugation according to the present invention has a cell membrane-penetrating peptide portion and a linking group portion for conjugating to a nucleic acid. The linking group portion contains at least one disulfide bond. Therefore, the nucleic acid conjugated to the cell membrane-penetrating peptide for conjugation according to the present invention is linked to the cell membrane-penetrating peptide via the linking group portion containing at least one disulfide bond.

Glutathione (GSH) is present in higher concentrations in cytoplasm than extracellular environment. For this reason, the nucleic acid conjugated to the cell membrane-penetrating peptide for conjugation according to the present invention not only has significantly improved cell penetration due to the cell membrane-penetrating peptide portion, but also is allowed to be present in a cell in a state of being separated into the nucleic acid portion and the cell membrane-penetrating peptide portion as a result of reductive cleavage of the disulfide bond by GSH. Generally, the structure and nature of nucleic acids modified with peptides are affected by the peptides, and therefore the originally targeted activity within the cell may decrease as compared to the case of unmodified nucleic acids. In contrast, by conjugating the introduction subject nucleic acid with the cell membrane-penetrating peptide for conjugation of the present invention, the introduction subject nucleic acid enjoys high cell penetration due to the cell membrane-penetrating peptide, while being allowed to be present in a free state without modification by the cell membrane-penetrating peptide, which enables the nucleic acid to exert its function within the cell to the same extent as a nucleic acid not modified by the cell membrane-penetrating peptide.

Specifically, the cell membrane-penetrating peptide for conjugation according to the present invention is represented by the following general formula (P1). In the general formula (P1), A¹ is a cell membrane-penetrating peptide, Z¹² is a divalent organic group, and q₂ is 0 or 1. When q₂ is 0, -(Z¹²)q₂- represents a single bond. In the cell membrane-penetrating peptide for conjugation according to the present invention, the cell membrane-penetrating peptide portion is -A¹, and the linking group portion is R¹⁰¹-S-S-(Z¹²)q₂-.

[Chem. 6] R¹⁰¹-S-S-(Z¹²)q₂-A¹ (P1)

### <Cell membrane-penetrating peptide>

In the general formula (P1), the cell membrane-penetrating peptide A¹, i.e., the cell membrane-penetrating peptide (hereinafter also referred to as the "cell membrane-penetrating peptide of the present invention") constituting the cell membrane-penetrating peptide portion of the cell membrane-penetrating peptide for conjugation according to the present invention, is a peptide with two or more amino acids, in which at least one of the amino acid residues constituting the peptide has, in its side chain, a C₁₋₃₀ alkyl group substituted with at least two fluorine atoms. When the C₁₋₃₀ alkyl group has two or more carbon atoms (i.e., in the case of a C₂₋₃₀ alkyl group), it may have 1 to 5 etheric oxygen atoms between the carbon atoms.

In the present invention and the present specification, "a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms" may be referred to as "Rf". That is, in the cell membrane-penetrating peptide according to the present invention, at least one of the amino acid residues constituting the peptide has Rf in the side chain.

In Rf, at least one of hydrogen atoms bonded to a carbon atom may further be replaced with a halogen atom other than a fluorine atom. The C₁₋₃₀ alkyl group in Rf is preferably a C₁₋₂₀ alkyl group, more preferably a C₁₋₁₀ alkyl group, further preferably a C₂₋₁₀ alkyl group, still more preferably a C₂₋₈ alkyl group. In Rf, the number of hydrogen atoms replaced with a fluorine atom is not particularly limited so long as it is 2 or more, and for example, preferably 3 or more, more preferably 6 or more, further preferably 7 or more.

Examples of Rf include a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group, a perfluorodecyl group, a difluoromethyl group, a 1,1-difluoroethyl group, a 2,2-difluoroethyl group, a 1,1,2,2-tetrafluoroethyl group, a 1,1,2,2,3,3-hexafluoropropyl group, a 1,1,2,3,3,3-hexafluoropropyl group, a 1,1,2,2,3,3-hexafluorohexyl group, a 1,1,2,2,3,3-hexafluorooctyl group, a 1,1,2,2,3,3-hexafluorodecyl group, a 1,1,2,2,3,3-hexafluorooctadecyl group, and a 1,1,2,2,3,3-hexafluorohexacosyl group.

When Rf is a group having 2 carbon atoms, Rf is preferably a group having at least 4 hydrogen atoms bonded to a carbon atom replaced with a fluorine atom, such as a pentafluoroethyl group, rather than a 1,1,1-trifluoroethyl group (CF₃-CH₂-). Further, when Rf is a group having 3 carbon atoms, Rf is preferably a linear group, and when it is a branched group, it is preferably a group having no or one trifluoromethyl group, rather than a group having two trifluoromethyl groups, such as a 1,1,1,3,3,3-hexafluoropropan-2-yl group ((CF3)₂-CH-). When Rf is a group having 4 carbon atoms, Rf is preferably a linear group, and when it is a branched group, it is preferably a group in which a hydrogen atom bonded to a carbon atom constituting the alkylene group moiety is replaced with a fluorine atom, or a perfluorinated group.

Rf is preferably a group represented by the following general formula (f-1) or (f-2). Rf^{P} means a perhalogenated C₁₋₁₀ alkyl group containing at least two fluorine atoms. Rf^{P} is a group in which all of hydrogen atoms in a C₁₋₁₀ alkyl group are replaced with halogen atoms, and at least two of such halogen atoms are fluorine atoms. When Rf^{P} has 2 or more carbon atoms, that is, when it is a perhalogenated C₂₋₁₀ alkyl group, it may have 1 to 5 etheric oxygen atoms between carbon atoms. In the present invention and the present specification, "a fully halogenated C₁₋₁₀ alkyl group containing at least two fluorine atoms (when the C₁₋₁₀ alkyl group has two or more carbon atoms, it may have an etheric oxygen atom between the carbon atoms)" means "a fully halogenated C₁₋₁₀ alkyl group containing at least two fluorine atoms, or a group having 1 to 5 etheric oxygen atoms between the carbon atoms of a fully halogenated C₂₋₁₀ alkyl group containing at least two fluorine atoms". In the general formula (f-2), the two Rf^{P} may be groups of the same type or different type.

In the following general formulae (f-1) and (f-2), n1 is an integer of from 0 to 10, and n2 is an integer of from 0 to 9. When n1 and n2 is 0, that indicates a single bond in either case. That is, when n1 is 0, the group represented by the general formula (f-1) is Rf^{P}-, and when n2 is 0, the group represented by the general formula (f-2) is (Rf^{P})₂-CH-.

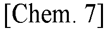

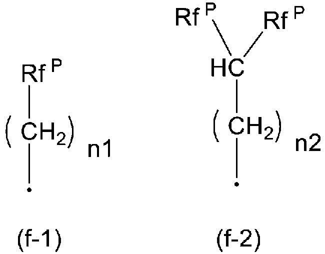

When Rf is a group represented by the general formula (f-1), Rf is preferably a group wherein Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n1 is an integer of from 0 to 4, more preferably a group wherein Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n1 is an integer of from 0 to 2, further preferably a group wherein Rf^{P} is a trifluoromethyl group, a pentafluoroethyl group, a heptafluoropropyl group, a nonafluorobutyl group, a perfluoropentyl group or a perfluorohexyl group, and n1 is an integer of from 0 to 2 (excluding a group wherein n1 is 1, and Rf^{P} is a trifluoromethyl group).

When Rf is a group represented by the general formula (f-2), Rf is preferably a group wherein Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n2 is an integer of from 0 to 4, more preferably a group wherein Rf^{P} is a trifluoromethyl group, a pentafluoroethyl group, a heptafluoropropyl group, a nonafluorobutyl group, a perfluoropentyl group, a perfluorohexyl group, a perfluoroheptyl group, a perfluorooctyl group, a perfluorononyl group or a perfluorodecyl group, and n2 is an integer of from 0 to 2, further preferably a group wherein Rf^{P} is a trifluoromethyl group, a pentafluoroethyl group, a heptafluoropropyl group, a nonafluorobutyl group, a perfluoropentyl group or a perfluorohexyl group, and n2 is an integer of from 0 to 2 (excluding a group wherein n2 is 0 or 1, and Rf^{P} is a trifluoromethyl group).

Of the groups represented by the general formula (f-1) and the general formula (f-2), Rf is preferably a group represented by the general formula (f-1), and more preferably a group represented by the general formula (f-1) in which n1 is 0. Of these, particularly preferable is a group represented by the general formula (f-1) in which n1 is 0 and two F atoms are bonded to the carbon atom closest to the bonding site of Rf^{P}, i.e., a group in which the site adjacent to the bonding site is -CF₂-.

Examples of Rf include a difluoromethyl group, a 1,1-difluoroethyl group, a 2,2-difluoroethyl group, a 1,1,2,2-tetrafluoroethyl group, a 1, 1,2,2,3,3-hexafluoropropyl group, and a 1,1,2,3,3,3-hexafluoropropyl group.

Examples of the cell membrane-penetrating peptide of the present invention include a peptide (Rf-containing peptide) containing at least one amino acid residue having Rf as its side chain. The number of such amino acid residue(s) is not particularly limited so long as at least one of the amino acid residues constituting the peptide has Rf as its side chain, and all the amino acid residues may have Rf as their side chains. When one molecule of the peptide has two or more amino acid residues having Rf as their side chains, the plurality of Rf may be of the same type or different types. Further, the amino acid residue having Rf as its side chain in the peptide may be on the N-terminal, may be on the C-terminal, or at a moiety other than the termini.

The cell membrane-penetrating peptide of the present invention may be a peptide consisting of two or more amino acids, and a peptide consisting of three or more amino acids is also preferred. The cell membrane-penetrating peptide of the present invention is preferably a peptide consisting of 2 to 40 amino acids, and more preferably a peptide consisting of 3 to 20 amino acids.

In the cell membrane-penetrating peptide of the present invention, the C-terminal or the N-terminal not linked to the linking group portion may be protected with a protecting group. As the protecting group for the C-terminal, groups listed below as examples of the protecting group R¹ for the carboxy group can be used, and a benzyl group is preferable. As the protecting group for the N-terminal, groups listed below as examples of the protecting group R² for the amino group can be used, and a Boc group or a Fmoc group are preferable.

In the cell membrane-penetrating peptide of the present invention, the amino acid residue having no Rf in its side chain is not particularly limited, and may be an α-amino acid residue, a β-amino acid residue, a γ-amino acid residue, or a δ-amino acid residue. The amino acid residue may also be a L-amino acid residue or a D-amino acid residue. The amino acid residue having no Rf in its side chain, which is contained in the cell membrane-penetrating peptide of the present invention, is preferably an amino acid residue of a protein-forming amino acid or its D forms, or its modified product (modified amino acid) with its side chain modified.

Examples of protein-forming amino acids include glycine, alanine, valine, leucine, isoleucine, serine, threonine, phenylalanine, tyrosine, tryptophan, asparagine, glutamine, proline, aspartic acid, glutamic acid, lysine, arginine, and histidine. Examples of modified amino acids obtained by modifying protein-forming amino acids include amino acids in which the hydrogen atom of the amino group in the side chain of lysine, arginine or histidine is replaced with any of groups listed for R² below or a Pbf (N-ω-(2,2,4,6,7-pentamethyldihydrobenzofuran-5-sulfonyl) group; amino acids in which the hydrogen atom of the carboxy group in the side chain of aspartic acid or glutamic acid is replaced with any of groups listed for R¹ below or an alkyl group such as a tert-butyl group; and amino acids in which the hydrogen atom of the thiol group of cysteine is replaced with a benzyl group.

Examples of the cell membrane-penetrating peptide of the present invention include tripeptides represented by the following general formulae (101-1), (101-2), (102-1), and (102-2). In the general formulae (101) and (102), R¹¹ and R¹² are each independently a C₁₋₆ alkyl group or a benzyl group, and are preferably each independently a methyl group or a benzyl group, and it is particularly preferred that R¹¹ is a methyl group and R¹² is a benzyl group. X is a hydrogen atom, Fmoc or Boc. Z is a C₁₋₆ alkoxy group, a hydroxy group, or an amino group. When Z is a C₁₋₆ alkoxy group, Z is particularly preferably a methoxy group. Further, a black dot means a binding site with the linking group.

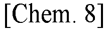

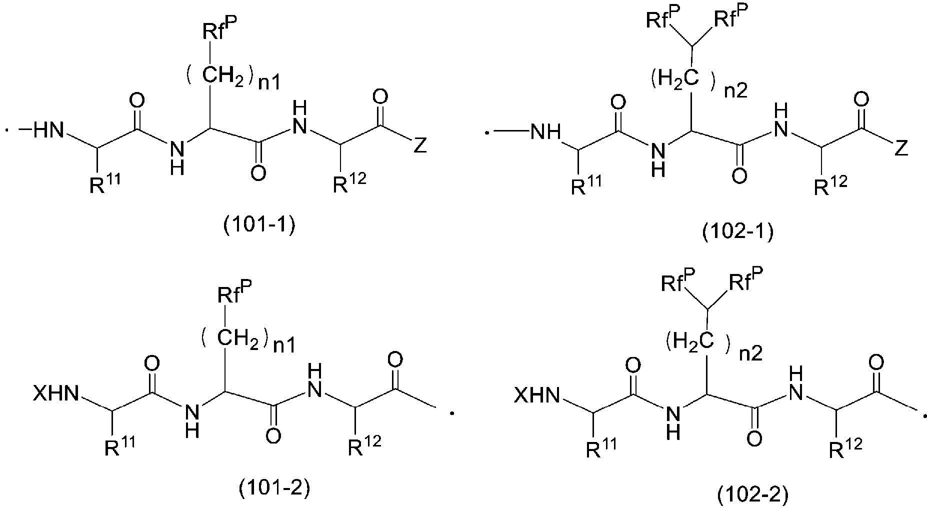

In the general formula (101-1), (101-2), (102-1) and (102-2), Rf^{P}, n1 and n2 are as defined for the general formulae (f-1) and (f-2). The group represented by the general formula (101-1), (101-2), (102-1), or (102-2) is preferably a group wherein Rf^{P} is a perfluorinated C1-10 alkyl group, and n1 or n2 is an integer of from 0 to 4, more preferably a group wherein Rf^{P} is a trifluoromethyl group, a pentafluoroethyl group, a heptafluoropropyl group, a nonafluorobutyl group, a perfluoropentyl group, a perfluorohexyl group, a perfluoroheptyl group or a perfluorooctyl group, and n1 or n2 is an integer of from 0 to 2, further preferably a group wherein Rf^{P} is a nonafluorobutyl group, a perfluoropentyl group, a perfluorohexyl group, a perfluoroheptyl group or a perfluorooctyl group, and n1 or n2 is an integer of from 0 to 2.

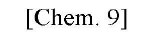

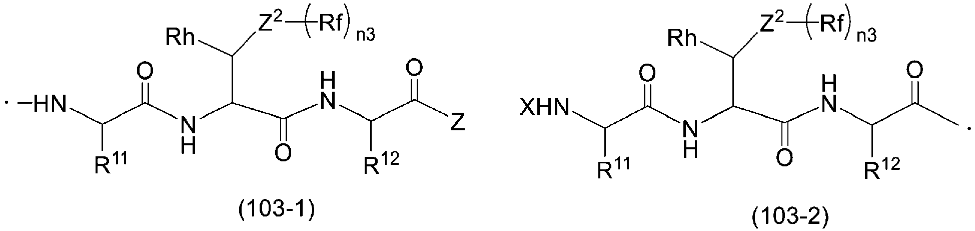

Examples of the cell membrane-penetrating peptide of the present invention include tripeptides represented by the general formulae (103-1) and (103-2). In the general formulae (103-1) and (103-2), Rf is as defined above, and n3 is 1, 2, or 3. Further, a black dot means a binding site with the linking group in the present invention.

In the general formulae (103-1) and (103-2), R¹¹, R¹², X, and Z are each as defined for the general formula (101-1), etc. In the tripeptide of the general formula (103-1) or (103-2), R¹¹ and R¹² are preferably each independently a methyl group or a benzyl group, and it is particularly preferable that R¹¹ is a methyl group and R¹² is a benzyl group.

In the general formulae (103-1) and (103-2), Rh is a hydrogen atom or a C₁₋₆ alkyl group. When Rh is a C₁₋₆ alkyl group, Rh is preferably a C₁₋₃ alkyl group, and more preferably a methyl group or an ethyl group.

In the general formulae (103-1) and (103-2), Z² is a divalent, trivalent, or tetravalent linking group other than an alkylene group. Z² is not particularly limited as long as it is a divalent, trivalent, or tetravalent group other than an alkylene group. For example, Z² can be an oxygen atom (-O-), a sulfur atom (-S-), -NH-, -N(CH₃)-, -N(C₂H₅)-, -N(C₃H₇)-, a trivalent nitrogen atom, -C(=O)-, -S(=O)₂-, a group in which 2 to 4 hydrogen atoms have been removed from a cycloalkane, a group in which 2 to 4 hydrogen atoms have been removed from an aromatic ring, a group in which 2 to 4 hydrogen atoms have been removed from a heterocycle, a group in which these are combined with an alkylene group, or a combination of these. The aryl group and heteroaryl group may be any of the groups listed above. However, an alkylene group itself or groups in which the linking portion to Rf is an alkylene group are excluded. Z² is preferably -C(=O)-, -C(=O)-O-, - O-C(=O)-, -NH-C(=O)-O-, -O-C(=O)-NH-, -C(=O)-NH-, -NH-C(=O)-, -S-S-, -S(=O)₂-NH-, -NH-S(=O)₂-, -S(=O)₂-NH-S(=O)₂-, or -C(=O)-NH-Ph- (-Ph- is a 1,4-phenylene group, a 1,3-phenylene group, a 1,5-phenylene group, or a 1,3,5-substituted phenyl group).

The cell membrane-penetrating peptide of the present invention can be produced by a general peptide synthesis method, for example, a solid-phase peptide synthesis method, except that an Rf group-containing amino acid, which is an amino acid having at least one Rf introduced into its side chain, is used as the raw material amino acid. The cell membrane-penetrating peptide of the present invention can be easily synthesized using an automatic peptide synthesizer from an amino acid having an Rf introduced into its side chain as the raw material.

A peptide can be produced in such a manner that amino acids having protected amino groups are sequentially condensed with an amino acid having its C-terminal bonded to a solid phase, and the resulting peptide is separated from the solid phase. The amino acid material is preferably one having its amino group protected with a Boc group or a Fmoc group. The amino acid material is preferably one having its side chain functional group protected with a protecting group. Examples of the protecting group for the side chain functional group include a Boc group, a triphenylmethyl group, a benzyl group, and a 2,2,5,7,8-pentamethylchroman-6-sulfonyl (Pmc) group.

Examples of a condensing agent for forming a peptide bond include N,N-dicyclohexylcarbodiimide (DCC), 1-ethyl-3-(3'-dimethylaminopropyl)carbodiimide (WSC), benzotriazol-1-yloxy-trisdimethylaminophosphonium hexafluorophosphate (BOP), benzotriazol-1-yloxytrispyrrolidinophosphonium hexafluorophosphate (pyBOP), 2-(1H-benzotriazol-1-yl)-1,1,3,3-tetramethyluronium hexafluorophosphate (HBTU), 2-(1H-benzotriazol-1-yl)-1,1,3,3-tetramethyluronium tetrafluoroborate, and 1-cyano-2-ethoxy-2-oxoethylideneaminooxy)dimethylaminomorpholinocarbenium hexafluorophosphate (COMU). Further, it is also possible to use a mixture of N-hydroxybenzotriazole (HOBt), ethyl (hydroxyimino)cyanoacetate (oxyma) and the above condensing agent that are used in a preferred ratio.

For formation of the peptide bond, a method that activates the carboxy terminal may be employed, and in such a case, the activating agent to be used may, for example, be N-hydroxysuccinimide, p-nitrophenylester or pentafluorophenylester. The base to be used when the peptide bond is formed, may, for example, be triethylamine or diisopropylethylamine (DIPEA). Examples of solvents used in the peptide bond forming reaction include chloroform, dichloromethane (DCM), dichloroethane (DCE), acetonitrile (ACN), N,N-dimethylformamide (DMF), dimethylsulfoxide (DMSO), and the like.

The Boc group and the Fmoc group, which are a protecting group for an amino-terminal amino group of the peptide or the amino acid, are respectively removed by trifluoroacetic acid and piperidine. The protecting group for the side chain functional group of the amino acid residue of the peptide may be removed, for example, by trifluoroacetic acid (TFA), hydrogen fluoride (HF) or trifluoromethanesulfonic acid.

Further, in the solid phase peptide synthesis method, as a method for separating a peptide or a peptide having a protecting group on the side chain functional group of the amino acid residue, from the solid phase peptide synthesis resin, for example, TFA may be used. Separation of the peptide from the solid phase peptide resin and removal of the protecting group on the side chain functional group of the amino acid residue, may be carried out simultaneously in the same reaction system. Alternatively, they may be carried out independently. Examples of the solid phase peptide synthesis resin for the solid phase peptide synthesis include commercially available products such as a 4-hydroxymethyl-3-methoxyphenoxy butyric acid/benzhydrylamine/polystyrene resin, a p-benzyloxy benzyl alcohol/polystyrene resin, and an oxime resin.

The desired peptide or its intermediate may be isolated and purified by various methods, such as ion chromatography, gel permeation chromatography, reversed phase chromatography, normal phase chromatography, recrystallization, extraction and fractional crystallization. Further, the peptide thus obtained may be converted into the corresponding salt by a conventional method.

The protecting group for an amino group or a carboxy group of the produced Rf-containing peptide may be removed by deprotection as the case requires. Deprotection may be conducted by a conventional method depending upon the type of the protecting group.

### <Reaction for synthesis of Rf-containing amino acid>

The Rf-containing amino acid can be produced, for example, by the following synthesis reaction.

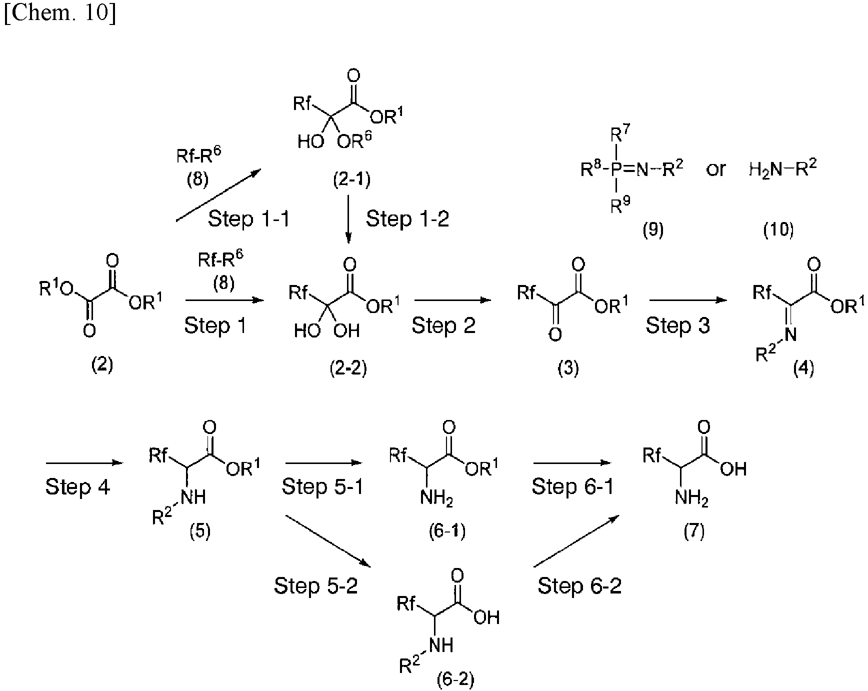

Rf is, specifically, preferably a group represented by the general formula (f-1) or (f-2) described below.

R¹ is a protecting group for a carboxy group and is specifically a protecting group selected form a group represented by the following general formula (p-1), a 2-(9,10-dioxo)anthrylmethyl group, a benzyloxymethyl group and a phenacyl group. In the general formula (p-1), R³ is a C₆₋₁₄ aryl group which may be substituted, and R⁴ and R⁵ are each independently a hydrogen atom or a C₆₋₁₄ aryl group which may be substituted. Further, the black dot means a bonding site.

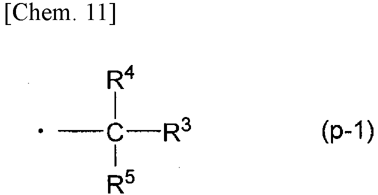

Examples of the protecting group for a carboxy group represented by R¹ a benzyl group, a diphenylmethyl group, a triphenylmethyl group, a 4-nitrobenzyl group, a 4-methoxybenzyl group, a 2,4-dimethoxybenzyl group, a 3,4-dimethoxybenzyl group, a 4-methylbenzyl group, a 2,6-dimethylbenzyl group, a 3-chlorobenzyl group, a 9-anthrylmethyl group, a piperonyl group, a 2-(9,10-dioxo)anthrylmethyl group, a benzyloxymethyl group and a phenacyl group. R¹ is preferably a benzyl group or a triphenylmethyl group, more preferably a benzyl group, which can be removed by deprotection under mild conditions.

The production method is advantageous in that by using an aralkyl protecting group such as a benzyl group or a triphenylmethyl group as the protecting group R1 for a carboxy group, R¹ can be removed by deprotection under mild conditions, and synthesis of the fluorinated amino acid and synthesis of the fluorinated peptide can be conducted without decomposing the functional group of the amino acid.

R⁶ is a silyl protecting group. Examples of R⁶ include a trimethylsilyl (TMS) group, a triethylsilyl (TES) group, a triisopropylsilyl (TIPS) group, a tertbutyldimethylsilyl (TBDMS) group and a tert-butyldiphenylsilyl (TBDPS) group. R⁶ is preferably a trimethylsilyl (TMS) group.

R² is a protecting group for an amino group. R² is not particularly limited so long as it is a protecting group for an amino group to be used for peptide synthesis. Examples of the protecting group for an amino group include a carbamate protecting group such as a tert-butoxycarbonyl (Boc) group, a 9-fluorenylmethyloxycarbonyl (Fmoc) group, a benzyloxycarbonyl (Cbz) group, an allyloxycarbonyl (Alloc) group and a 2,2,2-trichloroethoxycarbonyl (Troc) group. R² is preferably a tert-butoxycarbonyl (Boc) group or a 9-fluorenylmethyloxycarbonyl (Fmoc) group, which can be removed by deprotection under mild conditions.

### [Step 1]

By reacting the compound 2 and compound 8 in the presence of a metal fluoride, compound 2-2 can be obtained. The compound 8 represented by Rf-R⁶ of the general formula (8) can be synthesized from an easily available Rf-I (fluoroalkyl iodide) in one step, which allows a wide range of the Rf groups to be introduced.

As the metal fluoride, an alkali metal fluoride such as cesium fluoride, lithium fluoride or sodium fluoride may be used, and cesium fluoride is preferred.

The reaction may be conducted in a solvent inert to the reaction. The solvent may be an inert solvent such as tetrahydrofuran (THF), dichloromethane (DCM), acetonitrile, benzene, toluene, diethyl ether, 1,4-dioxane, N,N-dimethylformamide or N,N-dimethylacetamide, and is preferably tetrahydrofuran.

The amount of the compound 8 is preferably 0.5 to 10 mol per mol of the compound 2. The amount of the metal fluoride is preferably 0.01 to 2 mol per mol of the compound 2. The reaction of step 1 is conducted preferably at a temperature of 10°C or lower. By conducting the reaction at a temperature of 10°C or lower, the compound 2-2 can be produced with a high yield. The reaction temperature is preferably -78°C to 10°C, more preferably -50°C to -10°C, particularly preferably -40°C to -20°C. The reaction time is preferably 1 to 48 hours, and more preferably 6 to 36 hours.

The compound 2 may be produced by diesterifying oxalic acid by a known method, or it is also possible to use a commercial product.

### [Step 1-1]

In the reaction of step 1, compound 2-1 (a compound having one of hydroxy groups protected with R⁶), or a mixture of the compound 2-2 and the compound 2-1 may be obtained in some cases. In such cases, the silyl protecting group R⁶ for the compound 2-1 is removed by deprotection to obtain the compound 2-2.

The reaction of step 1-1 may be conducted in the same manner as in step 1.

### [Step 1-2]

By removing the silyl protecting group R⁶ for the compound 2-1 by deprotection, the compound 2-2 can be obtained.

Deprotection may be conducted in the presence of a fluoride such as tetrabutylammonium fluoride (TBAF), cesium fluoride or a hydrofluoride, or an acid such as hydrochloric acid, acetic acid or p-toluenesulfonic acid.

The reaction may be conducted in a solvent inert to the reaction. The solvent may be an inert solvent such as tetrahydrofuran, dichloromethane, acetonitrile, benzene, toluene, diethyl ether, 1,4-dioxane, N,N-dimethylformamide or N,N-dimethylacetamide, and is preferably tetrahydrofuran. It is preferred to add acetic acid.

With regard to the fluoride, the amount thereof per mol of the compound 2-1 (in the case of a mixture of the compound 2-2 and the compound 2-1, per mol of the mixture) is preferably 0.1 to 10 mol. With regard to the acid, the amount thereof per mol of the compound 2-1 (in the case of a mixture of the compound 2-2 and the compound 2-1, per mol of the mixture) is preferably 0.1 to 10 mol. The reaction of step 1-2 is conducted preferably at a temperature of 50°C or lower. By conducting the reaction at a temperature of 50°C or lower, the compound 2-2 can be produced with a high yield. The reaction temperature is preferably -80°C to 50°C, more preferably -40°C to 30°C, particularly preferably -20°C to 30°C. The reaction time is preferably 1 to 48 hours, and more preferably 6 to 36 hours.

### [Step 2]

By subjecting the compound 2-2 to dehydration reaction, compound 3 can be obtained.

The dehydration reaction may be conducted in the presence of a dehydrating agent such as diphosphorus pentoxide, concentrated sulfuric acid, calcium chloride, sodium sulfate, magnesium sulfate, calcium sulfate, molecular sieve (synthetic zeolite) or silica gel. The dehydrating agent is preferably diphosphorus pentoxide. The amount of the dehydrating agent is preferably 10 to 100 wt% relative to 100 wt% of the compound 2-2. The dehydrating reaction may be conducted by distilling the compound 2-2 in the presence of the dehydrating agent. Distillation is conducted preferably at a temperature of 30°C to 150°C. If the distillation temperature is too high, the compound 3 may decompose. If the distillation temperature is too low, the compound 3 may not be allowed to condense, resulting in a lower recovery rate. Distillation may be conducted under any pressure of reduced pressure, normal pressure and elevated pressure, and may properly be determined so that the boiling point of the compound 3 falls within the above preferred temperature range. The pressure is preferably 0.1 mmHg to 5 atm (3,800 mmHg).

### [Step 3]

By reacting the compound 3 with compound 9 or compound 10, compound 4 can be obtained.

In the general formula (9), R² is, as described above, a protecting group for an amino group. R⁷, R⁸ and R⁹ are each independently a C₆₋₁₄ aryl group. The C₆₋₁₄ aryl group represented by R⁷, R⁸ or R⁹ may, for example, be a phenyl group or a naphthyl group. R⁷, R⁸ and R⁹ are each preferably a phenyl group.

The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide, and is preferably diethyl ether.

The amount of the compound 9 or the compound 10 is preferably 0.5 to 10 mol per mol of the compound 3. The reaction temperature is preferably -78°C to 100°C, and more preferably 0°C to 40°C. The reaction time is preferably 1 minute to 24 hours, and more preferably 10 minutes to 4 hours.

In a preferred embodiment, by using a carbamate protecting group such as a tert-butoxycarbonyl group or a 9-fluorenylmethyloxycarbonyl group as the protecting group R² for an amino group, R² can be removed by deprotection under mild conditions, and synthesis of the fluorinated amino acid can be carried out while suppressing decomposition and racemization of the compound.

### [Step 4]

By subjecting the compound 4 to reduction reaction, compound 5 can be obtained.

The reduction reaction may be conducted by a method that uses a reducing agent or a method that performs reduction in the presence of a metal catalyst.

### (1) Method using reducing agent

As the reducing agent, a borohydride reagent such as sodium borohydride, zinc borohydride, sodium cyanoborohydride, lithium triethylborohydride, lithium tri(sec-butyl)borohydride, potassium tri(sec-butyl)borohydride, lithium borohydride or sodium triacetoxyborohydride may be used. The reducing agent is preferably sodium borohydride or zinc borohydride, more preferably sodium borohydride. The amount of the reducing agent is preferably 0.5 to 10 mol per mol of the compound 2.

The reaction may be conducted in a solvent inert to the reaction. The solvent may be an inert solvent such as diethyl ether, tetrahydrofuran, a hydrochlorofluorocarbon (HCFC) (for example, ASAHIKLIN (registered trademark) AK-225 (a mixture of 3,3-dichloro-1,1,1,2,2-pentafluoropropane and 1,3-dichloro-1,1,2,2,3-pentafluoropropane, AGC Inc.)), dichloromethane, acetonitrile, 1,4-dioxane, N,N-dimethylformamide, or N,N-dimethylacetamide, and is preferably diethyl ether.

The reaction temperature is preferably -78°C to 100°C, more preferably -10°C to 40°C. The reaction time is preferably 1 to 48 hours, and more preferably 6 to 36 hours.

### (2) Method performing reduction in the presence of metal catalyst

Examples of the metal catalyst include a palladium catalyst (such as palladium carbon, palladium hydroxide, Pearlman's catalyst, Lindlar's catalyst, silica gel-supported palladium catalyst, alumina-supported palladium catalyst or palladium oxide), a nickel catalyst (such as Raney nickel), a platinum catalyst (such as platinum carbon, platinum oxide, silica gel-supported platinum catalyst or alumina-supported platinum catalyst), a rhodium catalyst (such as rhodium carbon, alumina-supported rhodium catalyst or rhodium oxide), a ruthenium catalyst (such as ruthenium carbon, alumina-supported ruthenium catalyst or ruthenium oxide) and a cobalt catalyst (such as Raney cobalt), of which a palladium catalyst is preferable. The amount of the metal catalyst is preferably 0.0001 to 0.1 mol, and more preferably 0.0005 to 0.02 mol per mol of the compound 4.

The reaction may be conducted in a solvent inert to the reaction. The solvent may be an inert solvent such as methanol, ethanol, isopropanol, diethyl ether, tetrahydrofuran, ethyl acetate, dichloromethane, acetonitrile, 1,4-dioxane, N,N-dimethylformamide or N,N-dimethylacetamide.

The reduction reaction is conducted in the presence of hydrogen gas. The reduction reaction may be conducted under normal pressure or elevated pressure. The pressure of the hydrogen gas is preferably 0.5 atm to 10 atm. The reaction temperature is preferably 0°C to 100°C, and more preferably 10°C to 50°C. The reaction time is preferably 1 to 48 hours, and more preferably 6 to 36 hours.

### [Step 5-1]

By removing the protecting group R² for the compound 5 by deprotection, compound 6-1 can be obtained.

Deprotection may be conducted depending upon the type of the protecting group R².

When R² is a Boc group, deprotection may be conducted under acidic conditions. The acid used may, for example, be trifluoroacetic acid (TFA) or hydrochloric acid. The amount of the acid is preferably 1 to 1,000 mol per mol of the compound 5.

The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide, and is preferably dichloromethane or N,N-dimethylformamide. An acid may be used as the solvent. The solvent may be an inorganic acid such as hydrochloric acid, acetic acid or trifluoroacetic acid, or an organic acid, and is preferably trifluoroacetic acid. The reaction temperature is preferably -78°C to 50°C, and more preferably 0°C to 40°C. The reaction time is preferably 1 to 48 hours, and more preferably 6 to 36 hours.

When R² is a Fmoc group, deprotection may be conducted under basic conditions. The base to be used may be a secondary amine such as piperidine, morpholine or pyrrolidine. The amount of the base is preferably 1 to 100 mol per mol of the compound 5.

The reaction may be conducted in a solvent inert to the reaction. The solvent may be an inert solvent such as diethyl ether, tetrahydrofuran, dichloromethane, acetonitrile, benzene, toluene, 1,4-dioxane, N,N-dimethylformamide or N,N-dimethylacetamide. The reaction temperature is preferably -20°C to 80°C, and more preferably 0°C to 40°C. The reaction time is preferably 1 minute to 24 hours, and more preferably 5 minutes to 2 hours.

### [Step 6-1]

By removing the protecting group R¹ for the compound 6-1 by deprotection, compound 7 can be obtained.

Deprotection may be conducted depending upon the type of the protecting group R¹. When R¹ is a benzyl group, a triphenylmethyl group, a 9-anthrylmethyl group, a piperonyl group, a 2-(9,10-dioxo)anthrylmethyl group, a benzyloxymethyl group or a phenacyl group, deprotection may be carried out by a method of conducting reduction in the presence of a metal catalyst. The reduction reaction may be conducted in the same manner as the method of conducting reduction in the presence of a metal catalyst in step 4.

### [Step 5-2]

By removing the protecting group R¹ for the compound 5 by deprotection, compound 6-2 can be obtained. Deprotection may be conducted in the same manner as in step 6-1.

### [Step 6-2]

By removing the protecting group R² for the compound 6-2 by deprotection, compound 7 can be obtained. Deprotection may be conducted in the same manner as in step 5-1.

By conducting asymmetric reduction of the imine represented by the general formula (4) (compound 4), an optically active fluorine-containing amino acid (Rf-containing compound) can be synthesized. In the following reaction formula, the asterisk (*) means that the asymmetric carbon atom marked with the asterisk has an absolute configuration of S or R. Further, Rf, R¹ and R² are as defined above.

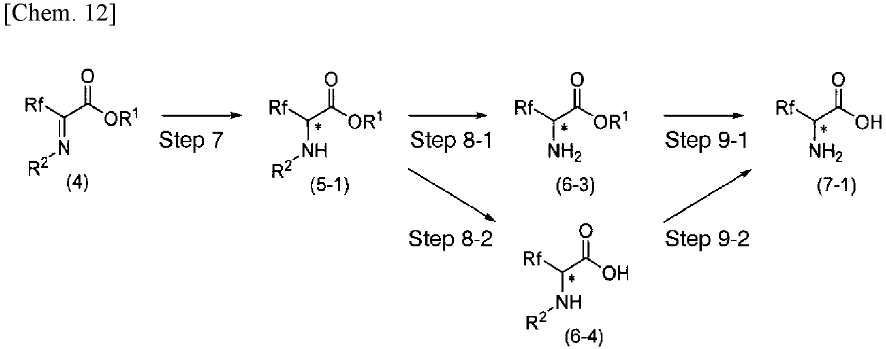

In the production method, use of an aralkyl protecting group such as a benzyl group or a triphenylmethyl group as the protecting group R¹ for a carboxy group, is advantageous in that R¹ can be removed by deprotection under mild conditions, and synthesis of the Rf-containing amino acid and synthesis of the Rf-containing peptide can be conducted while maintaining optical activity.

### [Step 7]

By subjecting the compound 4 to asymmetric reduction reaction, compound 5-1 can be obtained.

Asymmetric reduction reaction may be conducted by reducing the compound 4 in the presence of an asymmetric reduction catalyst.

As the asymmetric reduction catalyst, a transition metal complex having an asymmetric ligand coordinated to a transition metal may be used. Examples of the transition metal include palladium, rhodium, ruthenium, iridium, nickel, cobalt, platinum and iron. Examples of the transition metal complex include a palladium complex, a rhodium complex, a ruthenium complex, an iridium complex and a nickel complex.

The asymmetric ligand may be DPEN (1,2-diphenylethylenediamine), DAIPEN (1,1-di(4-anisyl)-2-isopropyl-1,2-ethylenediamine) or an optically active phosphine ligand. Examples of the optically active phosphine ligand include 2,2'-bis(diphenylphosphino)-1,1'-binaphthyl (BINAP), 2,2'-bis(diphenylphosphino)-5,5',6,6',7,7',8,8'-octahydro-1,1'-binaphthyl (H8-BINAP), 2,2'-bis(di-p-tolylphosphino)-1,1'-binaphthyl (Tol-BINAP), 2,2'-bis[bis(3,5-dimethylphenyl)phosphino]-1,1'-binaphthyl (Xyl-BINAP), 2,2'-bis[bis(3,5-di-tert-butyl-4-methoxyphenyl)phosphino]-1,1'-binaphthyl (DTBM-BINAP), 1,2-bis(anisylphosphino)ethane (DIPAMP), 2,3-bis(diphenylphosphino)butane (CHIRAPHOS), 1-cyclohexyl-1,2-bis(diphenylphosphino)ethane (CYCPHOS), 1,2-bis(diphenylphosphino)propane (PROPHOS), 2,3-bis(diphenylphosphino)-5-norbornene (NORPHOS), 2,3-O-isopropylidene-2,3-dihydroxy-1,4-bis(diphenylphosphino)butane (DIOP), 1-[1',2-bis(diphenylphosphino)ferrocenyl]ethylamine (BPPFA), 1-[1',2-bis(diphenylphosphino)ferrocenyl]ethyl alcohol (BPPFOH), 2,4-bis-(diphenylphosphino)pentane (SKEWPHOS), 1,2-bis(substituted phosphorano)benzene (DuPHOS), 5,5'-bis(diphenylphosphino)-4,4'-bi-1,3-benzodioxole (SEGPHOS), 5,5'-bis[di(3,5-xylyl)phosphino]-4,4'-bi-1,3-benzodioxole (DM-SEGPHOS), 5,5'-bis[bis(3,5-di-tert-butyl-4-methoxyphenyl)phosphino]-4,4'-bi-1,3-benzodioxole (DTBM-SEGPHOS), 1-[2-(2-substituted phosphino)ferrocenyl]ethyl-2-substituted phosphine (Josiphos), and 1-[2-(2'-2-substituted phosphinophenyl)ferrocenyl]ethyl-2-substituted phosphine (Walphos).

The amount of the asymmetric reduction catalyst is preferably 0.0001 to 0.1 mol, and more preferably 0.0005 to 0.02 mol per mol of the compound 4.

The reaction may be conducted in a solvent inert to the reaction. The solvent may be an inert solvent such as methanol, ethanol, isopropanol, diethyl ether, tetrahydrofuran, ethyl acetate, dichloromethane, acetonitrile, 1,4-dioxane, N,N-dimethylformamide or N,N-dimethylacetamide.

The reduction reaction is conducted in the presence of hydrogen gas. The reduction reaction may be conducted under normal pressure or elevated pressure. The pressure of the hydrogen gas is preferably 0.5 atm to 10 atm. The reaction temperature is preferably 0°C to 100°C, and more preferably 10°C to 50°C. The reaction time is preferably 1 to 48 hours, and more preferably 6 to 36 hours.

### [Step 8-1]

By removing the protecting group R² for the compound 5-1 by deprotection, compound 6-3 can be obtained. Deprotection may be conducted in the same manner as in step 5-1.

### [Step 9-1]

By removing the protecting group R¹ for the compound 6-3 by deprotection, compound 7-1 can be obtained. Deprotection may be conducted in the same manner as in step 6-1.

### [Step 8-2]

By removing the protecting group R¹ for the compound 5-1 by deprotection, compound 6-4 can be obtained. Deprotection may be conducted in the same manner as in step 6-1.

### [Step 9-2]

By removing the protecting group R² for the compound 6-4 by deprotection, the compound 7-1 can be obtained. Deprotection may be conducted in the same manner as in step 5-1.

Synthesis of the optically active fluorine-containing amino acid (Rf-containing compound) may also be conducted by the following reaction. In the following reaction formula, the asterisk means that the asymmetric carbon atom marked with the asterisk has an absolute configuration of S or R. Further, Rf, R¹ and R² are as defined above.

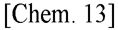

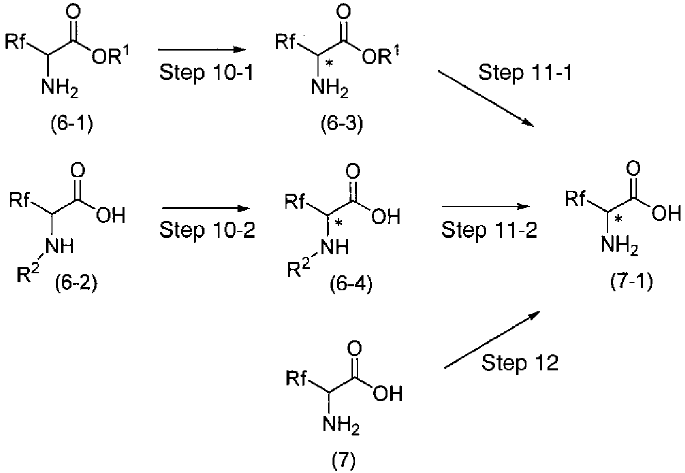

### [Step 10-1]

By subjecting the compound 6-1 to optical resolution, the compound 6-3 can be obtained.

The optical resolution may be conducted by a known means. For example, a method of using a chiral column, a method by crystallization or a diastereomer method may, for example, be used.

### (1) Method using chiral column

By liquid chromatography or supercritical fluid chromatography (SFC) using a chiral column, a racemic mixture may be resolved into optically active substances. As the chiral column, CHIRALPAK (registered trademark) (Daicel Corporation) and CHIRALCEL (registered trademark) (Daicel Corporation) may, for example, be used.

### (2) Method by crystallization

A salt of a racemic mixture and an optically active amine or an optically active acid is formed and is induced to a crystalline diastereomer salt, followed by fractional crystallization. Recrystallization is repeatedly carried out, which allows a single diastereomer salt to be obtained. As the case requires, the diastereomer salt is neutralized to obtain a free optically active substance. The optically active amine may, for example, be brucine, cinchonidine, cinchonine or 1-phenethylamine. The optically active acid may, for example, be camphorsulfonic acid, tartaric acid or mandelic acid.

### (3) Diastereomer method

An optically active reagent is reacted with the racemic mixture to obtain a diastereomer mixture, which is then subjected to fractional crystallization and chromatography to isolate a single diastereomer. From the obtained single diastereomer, the optically active reagent is removed to obtain the desired optical isomer.

### [Step 11-1]

By removing the protecting group R¹ for the compound 6-3 by deprotection, compound 7-1 can be obtained. Deprotection may be conducted in the same manner as in step 6-1.

### [Step 10-2]

By subjecting the compound 6-2 to optical resolution, the compound 6-4 can be obtained. Optical resolution may be conducted in the same manner as in step 10-1.

### [Step 11-2]

By removing the protecting group R² for the compound 6-4 by deprotection, the compound 7-1 can be obtained. Deprotection may be conducted in the same manner as in step 5-1.

### [Step 12]

By subjecting the compound 7 to optical resolution, the compound 7-1 can be obtained. Optical resolution may be conducted in the same manner as in step 10-1.

### <Method for producing Rf-containing peptide>

The Rf-containing peptide (the cell membrane-penetrating peptide of the present invention) can be produced using as a starting material an amino acid having Rf introduced into its side chain. For example, using the compound 6-1, the compound 6-2, the compound 6-3 or the compound 6-4 as the raw material, the Rf-containing peptide can be produced.

For example, the compound 6-2 or 6-4 is condensed with a fluorine-containing amino acid having its carboxy group protected, an amino acid having its carboxy group protected, a fluorine-containing peptide having its C-terminal protected, or a peptide having its C-terminal protected, whereby the Rf-containing peptide can be produced. Further, the compound 6-1 or the compound 6-3 is condensed with a fluorine-containing amino acid having its amino group protected, an amino acid having its amino group protected, a fluorinated peptide having its N-terminal protected, or a peptide having its N-terminal protected, whereby the Rf-containing peptide can be produced.

Further, the compound 7 or the compound 7-1, after having its amino group or carboxy group protected, may be subjected to condensation in the same manner to produce the Rf-containing peptide. Specifically, after the amino group is protected with a protecting group, the compound is condensed with a fluorine-containing amino acid having its carboxy group protected, an amino acid having its carboxy group protected, a fluorine-containing peptide having its C-terminal protected, or a peptide having its C-terminal protected. Alternatively, after the carboxy group is protected with a protecting group, the compound is condensed with a fluorine-containing amino acid having its amino group protected, an amino acid having its amino group protected, a fluorine-containing peptide having its N-terminal protected, or a peptide having its N-terminal protected.

### <Linking group portion>

In the general formula (P1), the divalent organic group of Z¹² is not particularly limited, and examples thereof include an alkylene group, an alkenylene group, an oxygen atom (-O-), a sulfur atom (-S-), -NH-, -N(CH₃)-, -N(C₂H₅)-, -N(C₃H₇)-, -C(=O)-, -S(=O)₂-, a polyethylene glycol group (PEG: -(C₂ H₄ O)n-), a siloxane bond, a silyl ether bond, a group with two hydrogen atoms removed from a cycloalkane, a group with two hydrogen atoms removed from an aromatic ring, a group with two hydrogen atoms removed from a heterocycle, sugar. Examples of the aromatic rings and the heterocycles include a pyrrole ring, a pyrazole ring, an imidazole ring, a triazole ring, a pyridine ring, a pyrimidine ring, a pyrazine ring, an oxazole ring, a thiazole ring, a furan ring, a thiophene ring, a benzene ring, and an indole ring. The alkylene group and alkenylene group may be linear, branched, or have a ring. As the divalent organic group or part thereof of Z¹², an alkylene group having 1 to 10 carbon atoms (C₁₋₁₀ alkylene group) or an alkenylene group having 2 to 10 carbon atoms (C₂₋₁₀ alkenylene group) are preferred. Further, these may be appropriately combined to form a part of the linking group of the present invention. Examples of such combined groups include -C(=O)-O-, -O-C(=O)-, -NH-C(=O)-O-, -O-C(=O)-NH-, -C(=O)-NH-, -NH-C(=O)-, -S(=O)₂-NH-, -NH-S(=O)₂-, -S(=O)₂-NH-S(=O)₂-, -C(=O)-NH-Ph- (-Ph - is a 1,4-phenylene group, a 1,3-phenylene group, a 1,5-phenylene group, or a 1,3,5-substituted phenyl group), -C₁₋₁₀ alkylene group-CO-, -C₂₋₁₀ alkenylene group-CO-, -C₁₋₁₀ alkylene group-NH-, and -C₂₋₁₀ alkenylene group-NH-.

In the general formula (P1), R¹⁰¹ is a 2-pyridyl group which may have a substituent. The substituent which the 2-pyridyl group may have is not particularly limited as long as it is a substituent which does not inhibit the nucleophilic substitution reaction of the sulfur atom belonging to the disulfide bond by a thiol derivative of nucleic acid. Specific examples of R¹⁰¹ include a 2-pyridyl group which has no substituent, a 3-nitro-2-pyridyl group, and the like.

For example, in the case of a compound of the general formula (P1) in which R¹⁰¹ is an unsubstituted 2-pyridyl group, q₂ is 1, and Z¹² is -CO- as -C₁₋₁₀ alkylene group, such a compound can be obtained by condensing the amino group or carboxyl group of the cell membrane-penetrating peptide of the present invention with a fatty acid residue having 2 to 11 carbon atoms, in which one hydrogen atom bonded to the carbon atom is replaced by a 2-pyridylthio group, or with a compound having an amino group in which one hydrogen atom bonded to the nitrogen atom is replaced by a (2-pyridylthio)-C₁₋₁₀ alkyl group or a (2-pyridylthio)-C₂₋₁₀ alkenyl group. As an example of a compound with 3 carbon atoms among examples of the "compound in which one hydrogen atom bonded to a carbon atom of a fatty acid having 2 to 11 carbon atoms is replaced with a 2-pyridylthio group", N-succinimidyl 3-(2-pyridylthio)propionate can be mentioned. Further, in the case of a compound of the general formula (P1) in which R¹⁰¹ is a 3-nitro-2-pyridyl group, q₂ is 1, and Z¹² is -C₁₋₁₀ alkylene group-NH-, such a compound can be obtained by condensing the amino group or carboxyl group of the cell membrane-penetrating peptide of the present invention with a fatty acid residue having 2 to 11 carbon atoms, in which one hydrogen atom bonded to the carbon atom is replaced with a 3-nitro-2-pyridylthio group, or a compound having an amino group in which one hydrogen atom bonded to the nitrogen atom is replaced with a (3-nitro-2-pyridylthio)-C₁₋₁₀ alkyl group or a (3-nitro-2-pyridylthio)-C₂₋₁₀ alkenyl group. As an example of a compound with 3 carbon atoms among examples of the "compound in which one hydrogen atom bonded to a carbon atom of a fatty acid having 2 to 11 carbon atoms is replaced with a 3-nitro-2-pyridylthio group", N-succinimidyl 3-(3-nitro-2-pyridylthio)propionate can be mentioned.

### <<Nucleic acid for transfection>>

The nucleic acid for transfection according to the present invention is a compound in which a subject nucleic acid to be introduced into a cell and a cell membrane-penetrating peptide are linked via a linking group containing at least one disulfide bond. The cell membrane-penetrating peptide can be the cell membrane-penetrating peptide of the present invention described above.

### <Introduction subject nucleic acid>

The introduction subject nucleic acid which is possessed by the nucleic acid for transfection according to the present invention is not particularly limited as long as it is a nucleic acid to be introduced into a cell. The introduction subject nucleic acid is preferably a functional nucleic acid that exhibits some physiological activity when taken up into a target cell in a living body. Specific examples include nucleic acids for expressing proteins in cells, nucleic acids for suppressing gene expression in cells, nucleic acids for performing gene modification, nucleic acid aptamers that specifically bind to target biomolecules, and functional nucleic acids that affect the physiological functions of cells. Examples of nucleic acids for expressing proteins include cDNA and mRNA that code for proteins, and may be expression plasmid vectors incorporating a region that codes for a protein of interest. Examples of nucleic acids for suppressing gene expression include functional nucleic acids used for RNA interference, such as siRNA, miRNA, shRNA, and antisense oligonucleotides, and may be RNAi vectors. Examples of nucleic acids for performing gene modification include fragments of genomic DNA. Examples of functional nucleic acids that affect the physiological functions of cells include decoy nucleic acids and CpG (cytosine-phosphate-guanine) oligonucleotides. Another example may be a nucleic acid having functional molecules such as fluorescent substances and functional peptides linked thereto. For example, the introduction subject nucleic acid may be a nucleic acid molecule in which a fluorescent substance is linked to the end of a single-stranded nucleic acid that has a guanine quadruplex (G-quadruplex) structure.

The introduction subject nucleic acid in the nucleic acid for transfection according to the present invention is not particularly limited, and may be a nucleic acid with all of the constituent nucleotides being natural nucleotides, or a nucleic acid with part or all of the constituent nucleotides being artificial nucleotides. Further, the introduction subject nucleic acid may be a single-stranded nucleic acid or a double-stranded nucleic acid. The introduction subject nucleic acid may be DNA, RNA, or a chimeric nucleic acid of DNA and RNA.

### <Linking group>

The linking group (hereinafter also referred to as the "linking group of the present invention") that links the introduction subject nucleic acid and the cell membrane-penetrating peptide in the nucleic acid for transfection according to the present invention is not particularly limited as long as it is an organic group that links the introduction subject nucleic acid and the cell membrane-penetrating peptide and has at least one disulfide bond at a position where the introduction subject nucleic acid and the cell membrane-penetrating peptide are separated by cleavage of the disulfide bond. The linking group of the present invention may be an organic group consisting only of a disulfide bond, or may be a group in which a disulfide bond is bonded to another di- or higher valent organic group. As the another divalent organic group, the same groups as listed above as examples of the divalent organic group for Z¹² in the cell membrane-penetrating peptide for conjugation of the present invention can be used.

By combining the cell membrane-penetrating peptide for conjugation according to the present invention with the subject nucleic acid to be introduced into the cell, represented by the following general formula (P2), the nucleic acid for transfection according to the present invention, represented by the following general formula (P3), can be produced.

[Chem. 14] B¹-(Z¹¹)q₁-SH (P2) B¹-(Z¹¹)q₁-S-S-(Z¹²)q₂-A¹ (P3)

In the general formula (P2), B¹ is a nucleic acid, Z¹¹ is a divalent organic group, and q₁ is 0 or 1. When q₁ is 0, -(Z¹¹)q₁- represents a single bond. As the divalent organic group of Z¹¹, the same groups as listed above as examples of the divalent organic group for Z¹² in the cell membrane-penetrating peptide for conjugation of the present invention can be used.

In the general formula (P3), A¹, Z¹², and q₂ are each as defined for the general formula (P1). In the formula (P3), B¹, Z¹¹, and q₁ are each as defined for the general formula (P2).

When the nucleic acid for transfection according to the present invention is represented by the general formula (P3), the nucleic acid for transfection according to the present invention separates into the introduction subject nucleic acid-(Z¹¹)q₁-SH and HS-(Z¹²)q₂-cell membrane-penetrating peptide by cleavage of the disulfide bond in the linking group. In the present invention, Z¹¹ is preferably an alkylene group having 1 to 6 carbon atoms, since the effect on the function of the introduction subject nucleic acid in the cell can be suppressed to low levels. Z¹² may be an appropriate combination of the organic groups listed above as the other divalent organic groups.

The method for transfection with a nucleic acid according to the present invention is a method that introduces the nucleic acid for transfection according to the present invention into a cell by contacting the nucleic acid according to the present invention with the cell. The cell membrane-penetrating peptide in the nucleic acid for transfection according to the present invention allows the nucleic acid for transfection to show superior cell penetration than a nucleic acid not linked to the peptide. Therefore, the nucleic acid for transfection according to the present invention can be introduced into cells by simply contacting the nucleic acid with the cells without using a general transfection reagent such as lipofectamine. When the target cell into which the nucleic acid for transfection according to the present invention is to be introduced is a cultured cell, the nucleic acid for transfection can be introduced into the cell by simply adding the nucleic acid for transfection to a culture medium and culturing the cell. In addition, for animal tissues, the nucleic acid for transfection according to the present invention can be introduced into the cells constituting the tissue by, for example, spraying or applying a solution of the nucleic acid for transfection according to the present invention onto the surface of the tissue. For this reason, for example, by producing the nucleic acid for transfection according to the present invention using a functional nucleic acid that exhibits some physiological activity when taken up into a target cell in a living body as the introduction subject nucleic acid, the efficiency of uptake of the functional nucleic acid into the target cell can be improved. In other words, by using the nucleic acid for transfection according to the present invention, a drug delivery system for delivering a nucleic acid drug into a cell can be easily constructed.

### [Examples]

Hereinbelow, the present invention will be described in more detail with reference to the Examples which, however, should not be construed as limiting the present invention.

The NMR apparatus used for analysis in Examples and Comparative Examples is JNM-ECZ400S (400 MHz) manufactured by JEOL Ltd. For ¹H NMR, the chemical shift of tetramethylsilane was assigned as 0 PPM, and for ¹⁹F NMR, the chemical shift of C6F6 was assigned as -162 PPM.

In the present specification, the following abbreviations are used.
Fmoc: 9-fluorenylmethyloxycarbonyl
C₆F₁₃: 1,1,2,2,3,3,4,4,5,5,6,6,6-tridecafluorohexyl
COMU: 1-cyano-2-ethoxy-2-oxoethylideneaminooxy)dimethylaminomorpholinocarbenium hexafluorophosphate
(CAS RN: 1075198-30-9)
oxyma: (hydroxyimino) cyanoethyl acetate (CAS RN: 3849-21-6)
DIPEA: Diisopropylethylamine

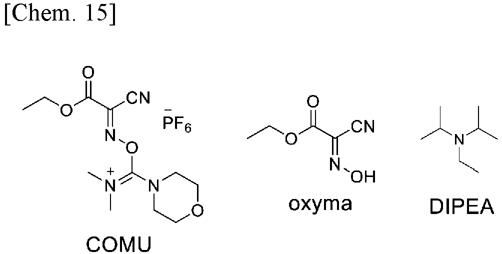

### <Cell culture>

In the following experiments, cell culture was performed as follows.

HeLa cells (Cell Bank of RIKEN) were cultured in low-glucose Dulbecco's Modified Eagle's Medium (D-MEM, manufactured by FUJIFILM Wako Pure Chemical Corporation) supplemented with 10% FBS and 0.5% penicillin/streptomycin in a humidified atmosphere (5% by volume CO₂) at 37°C.
24 hours before nucleic acid introduction, the cells were seeded in a 96-well culture plate (manufactured by Biolamo) at 1.0 x 10⁴ cells/well and allowed to adhere and grow (preculture).

### [Production Example 1]

### Synthesis of RF(C6)-containing amino acid (Fmoc-Asp(C₆F₁₃)-OH)

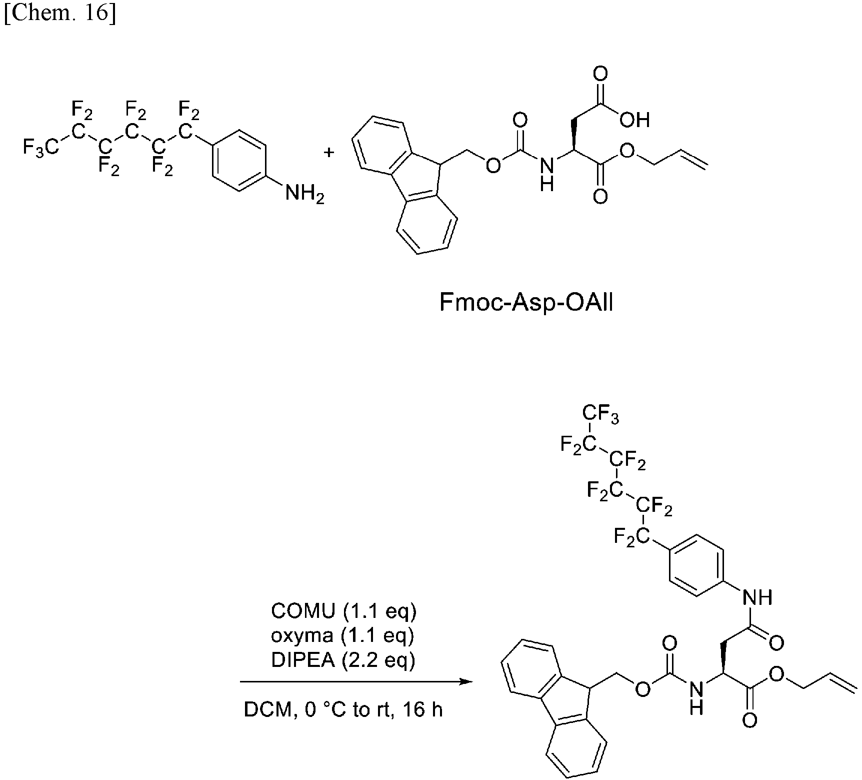

### 4-(perfluorohexyl)aniline was synthesized according to the method described in Org. Lett. , 2019, 21, 6481.

Further, in a dried 25 mL two-neck eggplant flask, 150 mg (0.3 mmol) of 4-(perfluorohexyl)aniline and 130 mg (0.33 mmol, 1.1 equivalents) of Fmoc-Asp-OAll were dissolved in 6 mL of dichloromethane, followed by addition of 141 mg (0.33 mmol, 1.1 equivalents) of COMU, 47 mg (0.33 mmol, 1.1 equivalents) of oxyma, and 85.3 mg (0.66 mmol, 2.2 equivalents) of DIPEA. The resulting was stirred at room temperature for 18 hours. Then, the resulting reaction mixture was quenched with HCl (1N) and extracted three times with dichloromethane. The added organic phase was concentrated under reduced pressure, diluted with ethyl acetate, and washed with HCl (1N), saturated aqueous sodium bicarbonate, and saturated saline. The washed organic phase was dried over sodium sulfate, filtered, and concentrated under reduced pressure to obtain a crude product of Fmoc-Asp(C₆F₁₃)-OAll. The crude product was dissolved in acetone and purified by reprecipitation with hexane to give pure Fmoc-Asp(C₆F₁₃)-OAll as a white solid (173 mg, 0.19 mmol, yield 64.9%).

### [Production Example 2]

### Synthesis of tripeptide H-Ala-Asp(C₆F₁₃)-Phe-NH₂

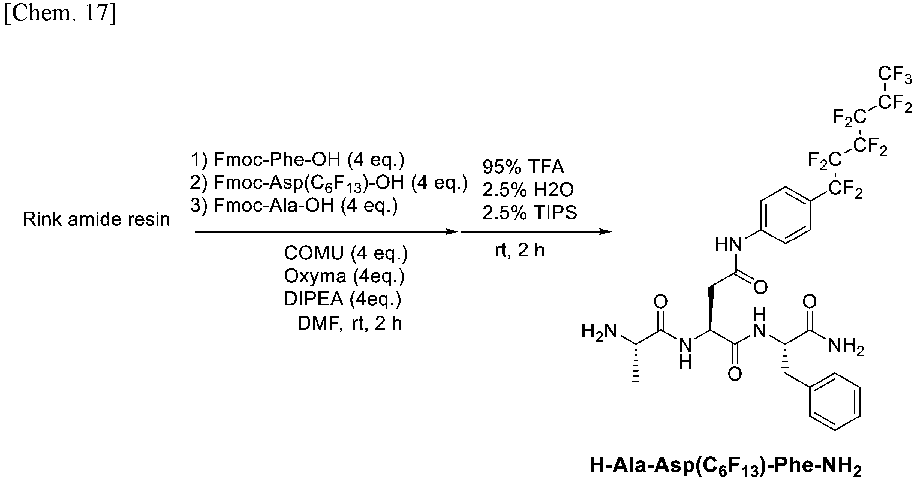

Rink-amide resin (39 mg, 0.025 µmol) and DMF (2 mL) were added to a solid-phase synthesis tube and the resulting was stirred at room temperature for 30 minutes to swell the resin. Next, a 20% piperidine/DMF solution (2 mL) was added to the tube and the resulting was stirred for 3 minutes, followed by washing three times with DMF (2 mL). A 20% piperidine/DMF solution (2 mL) was added again and the resulting was stirred for 12 minutes to carry out Fmoc group-deprotection. Next, Fmoc-Phe-OH (39 mg, 0.10 mmol, 4.0 equivalents), COMU (43 mg, 0.10 mmol, 4.0 equivalents), oxyma (14 mg, 0.10 mmol, 4.0 equivalents), and DIPEA (26 mg, 0.20 mmol, 8.0 equivalents) were added and the resulting was stirred at room temperature for 2 hours to carry out a condensation reaction of the amino acids. The reaction mixture was then washed three times with DMF (2 mL), and the Fmoc deprotection was carried out in the same manner as above. The same procedure was carried out for Fmoc-Asp(C₆F₁₃)-OH (84 mg, 0.10 mmol, 4.0 equivalents) and Fmoc-Ala-OH (31 mg, 0.10 mmol, 4.0 equivalents) synthesized in Production Example 1. As a subsequent post-treatment, a cleavage cocktail (TFA : triisopropylsilane : water = 95:2.5:2.5 (volume ratio)) (2 mL) was added and the resulting was stirred at room temperature for 2 hours to perform cleavage from the resin, yielding a crude product containing H-Ala-Asp(C₆F₁₃)-Phe-NH₂. The crude product was dried in vacuo and then purified by reverse phase chromatography (ACN/water/TFA = 75 : 25 : 0.1 to 9 : 9 : 1 : 0.1 (volume ratio) to give H-Ala-Asp(C₆F₁₃)-Phe-NH₂ (5 mg, 5 µmol, yield 20%).

### [Example 1]

A compound was produced in which a fluorescently labeled nucleic acid was linked to the tripeptide H-Ala-Asp(C₆F₁₃)-Phe-NH₂ via a disulfide bond.

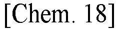

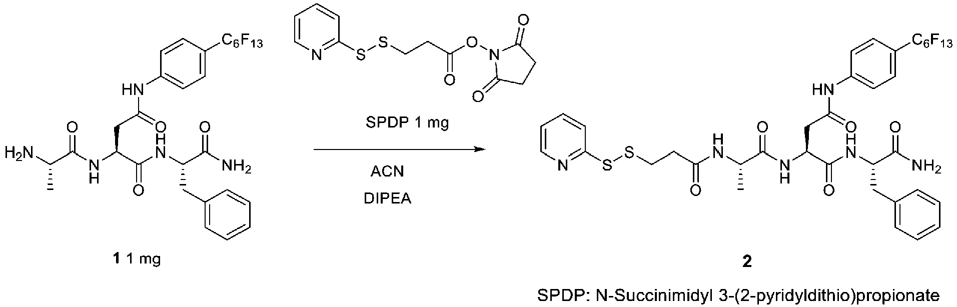

SPDP (N-succinimidyl 3-(2-pyridylthio)propionate) was condensed with the amino group of the tripeptide H-Ala-Asp(C₆F₁₃)-Phe-NH₂ (compound (1)) to obtain a compound (2).

Specifically, the compound (1) (1 mg), SPDP (1 mg), ACN (200 µL), and DIPEA (1 µL) were added and the resulting was stirred at room temperature for 18 hours. The resulting compound was passed through a 0.45 µm filter to remove insoluble matter, yielding a crude product.

The obtained crude product of the compound (2) was separated using reverse phase HPLC (60% ACN/40% H₂O-100% ACN (volume ratio)), and the purified compound (2) was confirmed by LC-MS.

### ESI-MS

### [M+H]+: m/z calculation for 941.18, found: 941.51 51

The entire amount of purified compound (2) was used to carry out a condensation reaction with a thiolated, FAM-modified DNA. The thiolated, FAM-modified DNA was prepared by linking a thiolhexylene group (i.e., a group in which the hydrogen atom bonded to the carbon atom at the end of the n-hexylene group is replaced with a thiol group) to the 5' end of DNA consisting of the base sequence of SEQ ID NO: 1 (TTTTTCAGTTGACCATATA) and linking FAM to the 3' end.

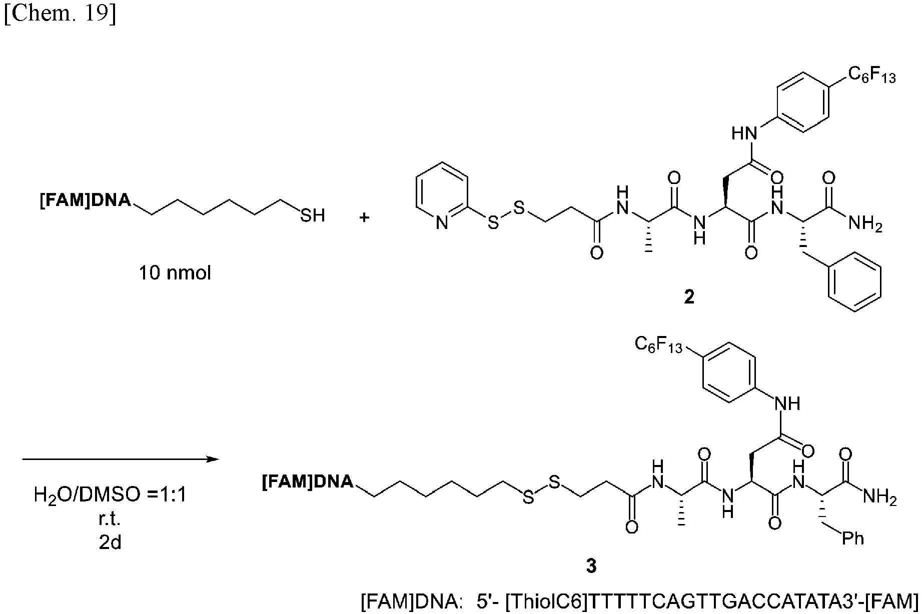

The entire amount of the purified compound (2) was mixed with a thiolated, FAM-modified DNA (10 nmol), and the resulting was dissolved in a mixed solvent of water and DMSO (water/DMSO = 1: 1 (volume ratio)), and reacted at room temperature for 2 days with stirring. The resulting reaction mixture was purified by reverse phase chromatography (ACN containing TEAA (10 mM)/water containing TEAA (10 mM) = 10:75 to 100:0 (volume ratio)) to obtain a compound (3) (yield 40.0% calculated by fluorimeter).

### MALDI-TOF MS [M]: m/z calcd for 7463, found: 7638

### [Test Example 1]

The efficiency of intracellular uptake of the compound (3) (FAM-modified DNA modified with tripeptide H-Ala-Asp(C₆F₁₃)-Phe-NH₂ (hereafter "CPP-DNA")) synthesized in Example 1 was examined. FAM-modified DNA (hereafter "control-DNA") was used as a control.

### <Preparation of sample solution>

A sample solution was prepared by adjusting the concentration of the control-DNA or CPP-DNA to 4 µM with OPTI-MEM culture medium. For blank measurements, OPTI-MEM liquid medium was used.

### <Evaluation of cell penetration by flow cytometry>

The medium of HeLa cells pre-cultured at 37°C for 24 hours was replaced with the sample solution, and the cell penetration was evaluated after culturing at 37°C for 4 hours. After culturing, the cell surface was washed three times with PBS (phosphate buffered saline), and the cells were detached and collected with Trypsin-EDTA 0.05% (manufactured by Gibco). The collected cells were then applied to a flow cytometer (GUAVA easyCyte (trademark) 8), and green 2 fluorescence (448 nm), which detects the fluorescent dye fluorescein (FAM) introduced into the synthetic nucleic acid, was measured.

The results of measuring the average fluorescence intensity in the flow cytometry for the cells are shown in FIG. 1. In FIG. 1, "control-DNA" indicates the result for the cells to which control-DNA was added to the medium, and "CPP-DNA" indicates the result for the cells to which FAM-modified nucleic acid (CPP-DNA) was added to the medium. As shown in FIG. 1, the cells to which CPP-DNA was added showed 1.6 times higher fluorescence intensity than the cells to which control-DNA was added. A T-test was performed, and it was shown that the observed difference in the amount of uptake between the CPP-DNA and the control-DNA was statistically significant.

### INDUSTRIAL APPLICABILITY

The present invention provides a nucleic acid for transfection in which a subject nucleic acid to be introduced into a cell and a cell membrane-penetrating peptide are linked via a disulfide bond. The nucleic acid for transfection according to the present invention can be efficiently introduced into a cell due to the cell membrane-penetrating peptide, but the function of the nucleic acid is fully exerted inside the cell as the nucleic acid is separated from the cell membrane-penetrating peptide. For this reason, the nucleic acid for transfection according to the present invention is expected to be used in the pharmaceutical field as a physiologically active substance, for example, as a carrier for introducing a gene medicine for gene modification or the like into target cells.

## Claims

1. A cell membrane-penetrating peptide for conjugation, represented by the following general formula (P1):
R¹⁰¹-S-S-(Z¹²)q₂-A¹ (P1)
in the general formula (P1), R¹⁰¹ is a substituted or unsubstituted 2-pyridyl group; A¹ is a cell membrane-penetrating peptide; Z¹² is a divalent organic group; and q₂ is 0 or 1,
wherein the cell membrane-penetrating peptide is a peptide with two or more peptide-bonded amino acids and with at least one of amino acid residues constituting the peptide having, in its side chain, a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms.

2. The cell membrane-penetrating peptide according to claim 1, wherein the side chain having the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is a group represented by the following general formula (f-1) or (f-2): 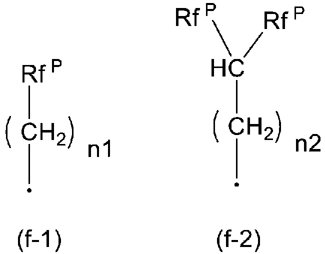 in the formulae, Rf^{P} is a fully halogenated C₁₋₁₀ alkyl group containing at least two fluorine atoms, or a group having 1 to 5 etheric oxygen atoms between carbon atoms of a fully halogenated C₂₋₁₀ alkyl group containing at least two fluorine atoms, n1 is an integer of 0 to 10, n2 is an integer of 0 to 9, and a black dot represents a bonding site.

3. The cell membrane-penetrating peptide according to claim 1, wherein the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is or is not further substituted with a halogen atom other than a fluorine atom.

4. The cell membrane-penetrating peptide according to claim 1, wherein a C-terminal or an N-terminal of the cell membrane-penetrating peptide is or is not protected with a protecting group.

5. The cell membrane-penetrating peptide according to claim 1, wherein the Z¹² is an alkylene group having 1 to 10 carbon atoms, an alkenylene group having 1 to 10 carbon atoms, an oxygen atom (-O-), a sulfur atom (-S-), -NH-, -N(CH₃)-, -N(C₂H₅)-, -N(C₃H₇)-, -C(=O)-, -S(=O)₂-, a poly(ethylene glycol) group (PEG: -(C₂ H₄ O)n-), a siloxane bond, a silyl ether bond, a group resulting from abstraction of two hydrogen atoms from a cycloalkane, a group resulting from abstraction of two hydrogen atoms from an aromatic ring, a group resulting from abstraction of two hydrogen atoms from a heterocycle, a sugar, or a group in which two or more of these are combined.

6. A method for producing a nucleic acid for transfection, comprising binding the cell membrane-penetrating peptide of any one of claims 1 to 5 to a target nucleic acid to be introduced into a cell to produce a nucleic acid for transfection, wherein the target nucleic acid is represented by the following general formula (P2):
B¹-(Z¹¹)q₁-SH (P2)
in the general formula (P2), B¹ is a nucleic acid; Z¹¹ is a divalent organic group; and q₁ is 0 or 1, and
the nucleic acid for transfection is represented by the following general formula (P3):
B¹-(Z¹¹)q₁-S-S-(Z¹²)q₂-A¹ (P3)
in the general formula (P3), A¹, Z¹², and q₂ are the same as in the general formula (P1); and B¹, Z¹¹, and q₁ are the same as in the general formula (P2).

7. A nucleic acid for transfection, comprising a subject nucleic acid to be introduced into a cell and a cell membrane-penetrating peptide that are linked via a linking group, wherein
the linking group contains at least one disulfide bond, and
the cell membrane-penetrating peptide is a peptide with two or more peptide-bonded amino acids and with at least one of amino acid residues constituting the peptide having, in its side chain, a C₁₋₃₀ alkyl group substituted with at least two fluorine atoms, or a group having 1 to 5 etheric oxygen atoms between carbon atoms of a C₂₋₃₀ alkyl group substituted with at least two fluorine atoms.

8. The nucleic acid for transfection according to claim 7, wherein the side chain having the C₁₋₃₀ alkyl group substituted with at least two fluorine atoms, or the group having 1 to 5 etheric oxygen atoms between carbon atoms of a C₂₋₃₀ alkyl group substituted with at least two fluorine atoms is a group represented by the following general formula (f-1) or (f-2): 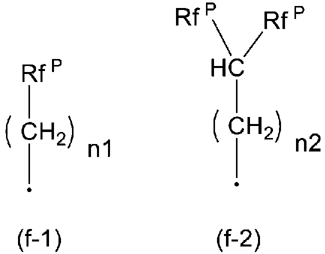 in the formulae, Rf^{P} is a fully halogenated C₁₋₁₀ alkyl group containing at least two fluorine atoms, or a group having 1 to 5 etheric oxygen atoms between carbon atoms of a fully halogenated C₂₋₁₀ alkyl group containing at least two fluorine atoms, n1 is an integer of 0 to 10, n2 is an integer of 0 to 9, and a black dot represents a bonding site.

9. The nucleic acid for transfection according to claim 7, wherein a C-terminal or an N-terminal of the cell membrane-penetrating peptide is or is not protected with a protecting group.

10. A method for transfection with a nucleic acid, comprising contacting the nucleic acid for transfection of any one of claims 7 to 9 with a cell to introduce the nucleic acid into the cell.
